# EUROPEAN PATENT APPLICATION

(11) **EP 3 085 365 A2**
(43) Date of publication of application: **26.10.2016**
(21) Application number: 16172113.9
(22) Date of filing: 19.01.2010
(51) Int. Cl.: A61K 31/01, A61K 31/015, A61K 31/05, A61K 31/07, A61K 31/12, A61K 31/192, A61K 31/225, A61K 31/352, A61K 45/06

(54) **SYNERGISTIC COMBINATIONS OF CAROTENOIDS AND POLYPHENOLS**

(30) Priority: 19.01.2009 US 145593 P; 04.12.2009 US 266517 P
(62) Divisional of application: 10731120.1
(71) Applicant: Lycored Ltd., 84102 Beer Sheva (IL)
(72) Inventor: Morris, Zelkha, 52232 Ramat-Gan (IL); Levy, Rachel, 84965 Omer (IL); Paran, Esther, 84965 Omer (IL); Yoav, Sharon, 84965 Omer (IL); Levy, Joseph, 84965 Omer (IL)
(74) Representative: Privett, Marianne Alice Louise

(57) **Abstract**

The present invention provides a therapeutic composition comprising one or more polyphenols and two or more carotenoids selected from the group consisting of lutein, lycopene and beta-carotene, wherein the polyphenols are selected from the group consisting of resveratrol, curcumin, quercetin, gallic acid, gingerol and chicoric acid. The invention also provides this composistion for use in inhibiting or reducing the production of superoxide ions, NO, TNF-alpha and/or PGE₂ or for treating an inflammatory condition in a mammalian subject.

## Description

### Field of the Invention

The present invention relates to a composition comprising synergistic combinations of polyphenols and carotenoids. More specifically, the present invention provides a composition. comprising a synergistic combination of the aforementioned compounds, which may be used *inter alia* to inhibit the production of various inflammatory mediators.

### Background of the Invention

The inflammatory process, which forms an important part of the non-specific immune system, is characterized by a complex set of chemical and cellular changes that are essential for host defense in the face of microbial agents and other potentially harmful environmental factors. However, in many cases, inflammation may be triggered inappropriately, and/or may persist to a degree which becomes harmful to the host. In such cases, there may be a need to inhibit or prevent the development of one or more aspects of the inflammatory process, in particular, in cases of non-infectious inflammatory diseases.

A very large number of different chemical mediators have been shown to be involved in the development and control of the inflammatory process. Recent studies by a number of different laboratories have implicated nitric oxide (NO) as an important modulator of a variety of acute and chronic inflammatory disorders, including various types of arthritis, gastrointestinal diseases, inflammatory conditions of the central nervous system and certain forms of asthma. Consequently, it has been proposed that inhibition of NO production could provide a useful therapeutic mechanism for the treatment and/or management of these inflammatory disorders. Furthermore, inhibition of NO. synthesis has also been shown to be useful in some conditions or states that are not primarily inflammatory in nature. Thus, for example, inhibition of NO synthesis has been found to reduce glucose uptake into limb tissue in individuals with Type 2 diabetes during exercise.

The *in vivo* production of NO is mediated by a' family of nitric oxide synthase (NOS) enzymes, including inducible-nitric oxide synthase (I-NOS), which is activated by many different immunological stimuli including lipopolysaccharide (LPS), interferon gamma and interleukin 1 (IL-1).

Inhibition of NOS may be achieved both in *vitro* and *in vivo* by the use of L-N^{G}-monomethyl Arginine citrate (L-NMMA). In addition, several other compounds, including a number of natural products, have also been shown to inhibit NO production. The latter group includes compounds such as lutein *[*Rafi M.M. et al. Mol Nutr Food Res. 2007 Mar;51(3):333-40*;* Choi, J.S. Nutrition. 2006 Jun;22 (6) : 668-71) and lycopene [Rafi, M.M. et al. J Food Sci. 2007 Jan;72(1):S069-74*].* However, the efficacy and potency of many of the natural product NO inhibitors have proven to be not particularly high. A need therefore exists for improved NO production-inhibiting compositions of natural origin.

Another highly important inflammatory mediator is the tumor necrosis factor-alpha (TNF-alpha), which is a cytokine produced by a variety of cell types including macrophages, neutrophils and lymphocytes. TNF-alpha occupies a key position in the early stage of the inflammatory process and is responsible for stimulating the production of other factors such as nuclear factor κB which in turn causes activation of a wide range of pro-inflammatory genes. Thus, in view of its key pro-inflammatory role, TNF-alpha is clearly an important potential therapeutic target for anti-inflammatory agents.

A third key inflammatory mediator is prostaglandin E₂ (PGE₂), a member of the eicosanoid family of regulatory molecules. Thus, PGE₂ is produced in significant amounts at inflammatory sites, where it acts as a vasodilator, and also (together with other mediators such as histamine and bradykinin) causes an increase in vascular permeability, thereby contributing to most of the classical signs of inflammation.

Finally, another pro-inflammatory mediator that is released by inflammatory cells such as macrophages and neutrophils is the superoxide ion. While superoxide ions are highly effective in killing microbial invaders, in other (particularly non-infective) inflammatory conditions, these ions may cause extensive host. tissue damage. The production of superoxide ions is therefore a potentially useful therapeutic target when considering new means for controlling inflammatory states.
It is a purpose of the present invention to provide a composition that may be used to inhibit the production of one or more key inflammatory mediators, such as superoxide ions, NO, TNF-alpha and/or PGE₂, as a means for treating or managing pathological states and processes in which said mediators are implicated.

It is another purpose of the invention to provide a composition that is able to inhibit the production of the aforesaid inflammatory mediators with greater efficacy and/or potency than the compounds and compositions reported in the prior art.

### Summary of the Invention

It has now been unexpectedly found by the present inventors that polyphenol compounds may synergistically interact with carotenoids in the inhibition several pro-inflammatory pathways. In particular, it has now been found that the polyphenol compound carnosic acid causes synergistic enhancement of the inhibitory effect of certain carotenoids such as lycopene, lutein and beta-carotene on the production of the inflammatory mediators NO, TNF-alpha and PGE₂. Furthermore, while this synergistic effect is seen in binary combinations of carnosic acid together with lycopene, beta-carotene or lutein, the synergism is significantly greater when carnosic acid is combined with two of the aforementioned carotenoids. The aforementioned synergistic anti-inflammatory effect is also seen when the carotenoids are present in combination with other polyphenols such as quercetin, resveratrol and gallic acid.

The present invention is therefore primarily directed to a therapeutic composition comprising one or more polyphenols and two or more carotenoids selected from the group consisting of lutein, lycopene and beta-carotene.

In one preferred embodiment, the polyphenols used in the compositions of the present invention are selected from the group consisting of carnosic acid, quercetin, resveratrol, gallic acid, chicoric acid, gingerol and curcumin.

In one particularly preferred embodiment, the compositions of the present invention comprise the polyphenol compound carnosic acid.

In another particularly preferred embodiment, the compositions of the present invention comprise the polyphenol compound quercetin.

In another particularly preferred embodiment, the compositions of the present invention, comprise the polyphenol compound resveratrol.

In another particularly preferred embodiment, the compositions of the present invention comprise the polyphenol compound gallic acid.

In one embodiment, the aforementioned therapeutic composition comprises carnosic acid, lycopene and lutein.

In another preferred embodiment, the composition comprises carnosic acid, lutein and beta-carotene.

In a still further preferred embodiment, the composition comprises lycopene, beta carotene and carnosic acid.

In another embodiment, the composition consists essentially of lycopene, lutein and carnosic acid.

In a further preferred embodiment, the composition consists essentially of lutein, beta-carotene and carnosic acid.

In a still further preferred embodiment, the composition consists essentially of lycopene, beta-carotene and carnosic acid.

It is to be noted that the term "consists essentially of", as .used throughout this disclosure and appended claims refers to the situation wherein the composition of the present invention may comprise, in addition to the named elements (i.e. carnosic acid together with lycopene and/or lutein), other compounds, substances and agents which do not materially affect the basic and novel characteristics of the present invention.

In other preferred embodiments, the compositions of the above-disclosed preferred embodiments may further comprise one or more additional carotenoids. In one particularly preferred embodiment, the additional carotenoids are selected from the group consisting of phytoene and phytofluene.

The active components of the above-disclosed compositions (i.e. polyphenol(s) and carotenoids) may be purified compounds, synthetic compounds or may be present in mixture with other components, for example in plant extracts such as rosemary extract (in the case of carnosic acid), marigold extract (in the case of lutein) or a tomato extract (such as Lycomato - which is commercially available from LycoRed, Be'er Sheva, Israel - in the case of lycopene and other carotenoids).

It should be noted that the term "lutein" as used in the present disclosure should be understood to include all lutein esters within its scope. In addition, the term "lutein" may also be taken to include within its scope a mixture of lutein and zeaxanthin, since the last-mentioned carotenoid is often present together with lutein (sometimes constituting 0.1% - 15%, and more often 4%-6% of the lutein content).

In another aspect, the present invention provides a method for inhibiting or reducing the production of superoxide ions, NO, TNF-alpha and/or PGE₂ in a mammalian subject comprising administering to said subject a therapeutic composition according to any of the embodiments disclosed hereinabove.

Furthermore, the present invention also provides a method of treatment of pathological conditions in which superoxide ions, NO, TNF-alpha and/or PGE₂ acts as a modulator or mediator of said condition in a mammalian subject in need of such treatment, wherein said method comprises administering to said subject a therapeutic composition according to any one of the embodiments disclosed hereinabove. In one preferred embodiment of this method, the condition to be treated is selected from the group consisting of acute inflammatory conditions, chronic inflammatory conditions, rheumatoid arthritis, adult respiratory distress syndrome (ARDS), asthma, rhinitis, idiopathic pulmonary fibrosis, peritonitis, cardiovascular inflammation, myocardial ischemia, reperfusion injury, atherosclerosis, sepsis, trauma, diabetes type II, retinopathy, psoriasis, gastrointestinal inflammation, cirrhosis, peritonitis and inflammatory bowel disease, and neurodegenerative diseases, such as for example Alzheimer's disease (AD).

In particularly preferred embodiments of the methods described hereinabove, the mammalian subject is a human subject.

While in the above-disclosed methods, the therapeutic composition may be administered by any convenient means, in one preferred embodiment said composition is administered in a pharmaceutical dosage form. In another preferred embodiment, however, the therapeutic composition is incorporated into a foodstuff or beverage.

In another aspect, the present invention is directed to the use of a combination of one or more polyphenols and two or more carotenoids selected from the group consisting of lycopene, beta-carotene and lutein in the manufacture of a medicament for the treatment of conditions responsive to inhibition of NO, TNF-alpha and/or PGE₂ production.

In one preferred embodiment of this aspect of the invention, the one or more polyphenols are selected from the group consisting of carnosic acid, quercetin, resveratrol, gallic acid, chicoric acid, gingerol and curcumin.

In one particularly preferred embodiment of this aspect of the invention, the polyphenol is carnosic acid.

In another particularly preferred embodiment of this aspect of the invention, the polyphenol is quercetin.

In yet another particularly preferred embodiment of this aspect of the invention, the polyphenol is resveratrol.

In yet another particularly preferred embodiment of this aspect of the invention, the polyphenol is gallic acid.

In one preferred embodiment, the condition to be treated is an inflammatory condition.

In one preferred embodiment of the above-disclosed use, the condition to be treated is selected from the group consisting of acute inflammatory conditions, chronic inflammatory conditions, rheumatoid arthritis, adult respiratory distress syndrome (ARDS), asthma, rhinitis, idiopathic pulmonary fibrosis, peritonitis, cardiovascular inflammation, myocardial ischemia, reperfusion injury, atherosclerosis, sepsis, trauma, diabetes type II, retinopathy, psoriasis, gastrointestinal inflammation, cirrhosis, peritonitis and inflammatory bowel disease, and neurodegenerative diseases, such as for example Alzheimer's disease (AD).

In one particularly preferred embodiment of this aspect of the invention, carnosic acid is used in combination with both lycopene and lutein.

In another particularly preferred embodiment of this aspect of the invention, carnosic acid is used in combination with both lycopene and beta-carotene.

In a still further preferred embodiment of this aspect of the invention, carnosic acid is used in combination with both lutein and beta-carotene.

All the above and other characteristics and advantages of the present invention will be further understood from the following illustrative and non-limitative examples of preferred embodiments thereof.

### Brief Description of the Drawings

**Fig. 1** graphically depicts the synergistic interaction of carnosic acid and lycopene in the inhibition of NO production by peritoneal macrophages. The upper panel shows the results obtained with purified lycopene, while the lower panel presents the results obtained with a lycopene-rich tomato extract.
**Fig. 2a** graphically illustrates the synergistic interaction between carnosic acid and purified lycopene (upper graphs) and between carnosic acid and a lycopene-rich tomato extract (lower graph) in the inhibition of NO production by peritoneal macrophages.
**Fig. 2b** graphically illustrates the synergistic interactions between lycopene and various combinations of lutien, carnosic acid and beta-carotene in the inhibition of NO production by peritoneal macrophages. The upper graph shows the results obtained with purified lycopene, while the lower graph presents the results obtained with a lycopene-rich tomato extract.
**Fig. 2c** further illustrates the synergistic interactions between lycopene and various combinations of lutein, carnosic acid and beta-carotene in the inhibition of NO production by peritoneal macrophages. The upper graph shows the results obtained with purified lycopene, while the lower graph presents the results obtained with a lycopene-rich tomato extract.

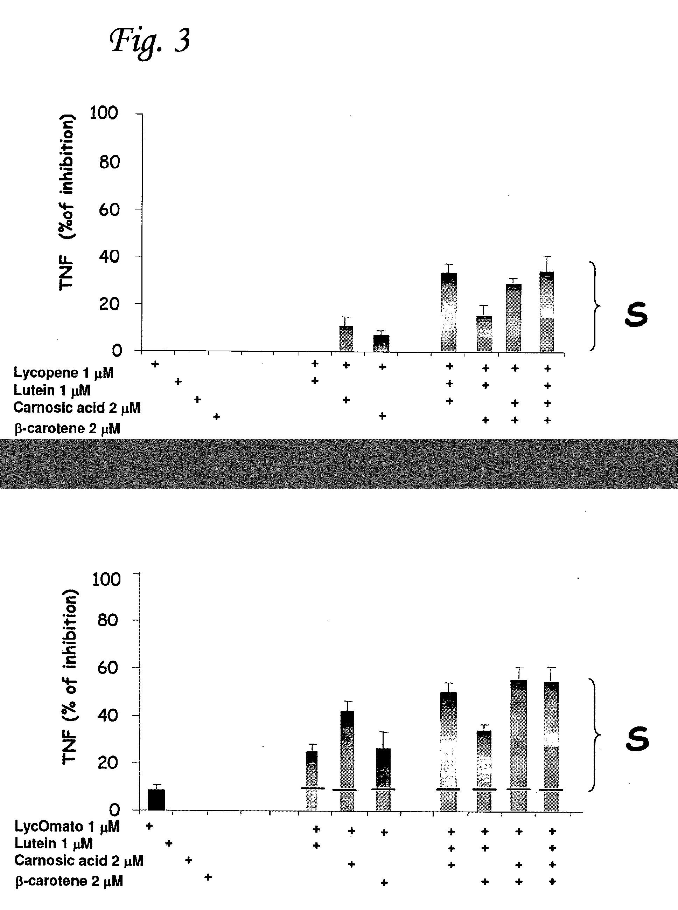

**Fig. 3** graphically illustrates the synergistic interactions between lycopene and various combinations of lutein, carnosic acid and beta-carotene in the inhibition of TNF-alpha production by peritoneal macrophages. The upper graph shows the results obtained with purified lycopene, while the lower graph presents the results obtained with a lycopene-rich tomato extract.
**Fig. 4** graphically illustrates the synergistic interactions between lycopene and various combinations of lutein, carnosic acid and beta-carotene in the inhibition of PGE₂ production by peritoneal macrophages, in comparison to the non synergistic effect of combinations excluding lycopene.
**Fig. 5a** graphically illustrates the synergistic interactions between purified lycopene and various combinations of different mixtures of lutein, carnosic acid and beta-carotene in the inhibition of PGE₂ production by peritoneal macrophages.
**Fig. 5b** graphically illustrates the synergistic interactions between a lycopene-rich tomato extract and various combinations of different mixtures of lutein, carnosic acid and beta-carotene in the inhibition of PGE₂ production by peritoneal macrophages.
**Fig. 6** graphically illustrates the synergistic interactions between lutein, beta-carotene and carnosic acid in the inhibition of LPS-stimulated NO production by peritoneal macrophages.
**Fig. 7** graphically illustrates the synergistic interactions between lutein, beta-carotene and carnosic acid in the inhibition of LPS-stimulated TNFα production by peritoneal macrophages.
**Fig. 8** graphically illustrates the synergistic interaction between lycopene, lutein and various polyphenols in the inhibition of LPS-stimulated NO production by peritoneal macrophages. Panel A presents the results using purified lycopene, while panel B presents the results obtained using a lycopene-containing tomato extract (Lyc-O-Mato).

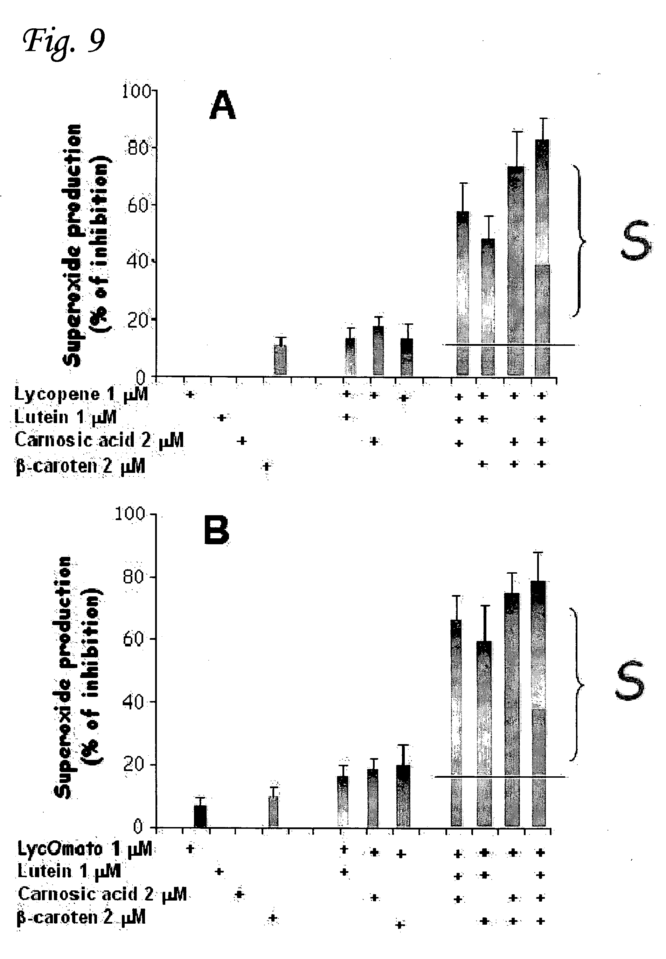

**Fig. 9** graphically illustrates the synergistic interaction between lycopene, lutein, beta-carotene and carnosic acid on the inhibition of macrophage superoxide production. Panel A presents the results using purified lycopene, while panel B presents the results obtained using a lycopene-containing tomato extract (Lyc-O-Mato)
**Fig. 10** demonstrates the synergistic interaction between lycopene-or Lyc-O-Mato with lutein and carnosic acid on the inhibition of p65-NFkB phosphorylation on Serine 536 in cell nuclear lysates, following a 10 minute preincubation with LPS. The upper portion of the figure presents the immunoblot results from which the graphical data were derived.
**Fig. 11** graphically illustrates the synergistic interaction between lycopene or Lyc-O-Mato with lutein and carnosic acid on the inhibition of LPS-inducible nitric oxide synthase (iNOS) and of cyclooxygenase 2 (COX2) protein expression in total cell lysates.

### Detailed Description of Preferred Embodiments

As disclosed hereinabove, the present invention provides compositions comprising combinations of one or more polyphenols with one or more carotenoids. In a particularly preferred embodiment of the invention, the compositions comprise carnosic acid as the sole polyphenol and one or more carotenoids selected from the group consisting of lycopene (either purified or contained within a tomato extract), lutein and beta-carotene. In other preferred embodiments, the sole polyphenol component is selected from the group consisting of quercetin, resveratrol and gallic acid.

Preferred daily amounts of each of the active agents present in the compositions that are administered to subjects in need of such administration are as follows:
Carnosic acid: 0.5 to 30 mg
Lycopene: 0.5 to 30 mg
Lutein: 0.5 to 30 mg
Beta-carotene: 0.5 to 30 mg

More preferably, the daily amount of each of the aforementioned active agents is in the range of 1 to 5 mg.

The amount of each of the various active components may be selected such that the weight ratios therebetween fall within the following broad range:
Lycopene : Lutein : Beta-carotene : Carnosic acid 0.1-5.0 : 0.1-5.0: 0.1-5.0 : 0.1-5.0

In one preferred group of compositions, the active components may be combined in the following weight ratio ranges:
Lycopene : Lutein : Beta-carotene : Carnosic acid 0.1-1.0 : 0.1-1.0: 0.1-1.0 : 0.1-1.0

In one preferred embodiment, the active components may be combined in the following ratio:
Lycopene : Lutein : Beta-carotene : Carnosic acid 1.0 : 1.0 : 1.0 : 0.5

In another preferred embodiment, the active components may be combined in the following ratio:
Lycopene : Lutein : Beta-carotene : Carnosic acid 1.0 : 0.3 : 0.3 : 0.4

In a still further preferred embodiment, the active components may be combined in the following ratio:
Lycopene : Lutein : Beta-carotene : Carnosic acid 1.0 : 1.0 : 1.0 : 1.0

It is to be noted that the compositions prepared in accordance with the preceding examples of preferred weight ratios do not require the obligatory presence of all four components listed. Rather, it is sufficient for the composition to comprise carnosic acid (or another polyphenol) together with at least two of the indicated carotenoids, wherein the relative amount of each of these components is as indicated by the figures provided immediately hereinabove.

In another group of preferred embodiments, the active components may be combined in the following weight ratio ranges:
Lycopene : Lutein : Beta-carotene : Carnosic acid 0.1-1.0 : 0.1-5.0: 0.1-1.0 : 0.1-1.0

More preferably, the active components may be combined in the following weight ratio ranges:
Lycopene : Lutein : Beta-carotene : Carnosic acid 0.1-1.0 : 1.0-4.0: 0.1-1.0 : 0.1-1.0

In specific preferred embodiments, the active components may be combined in the following weight ratios:
Lycopene : Lutein : Carnosic acid 0.1 : 1.73 : 0.13
Lycopene : Lutein : Carnosic acid 0.1 : 1.8 : 0.26
B-carotene : Lutein : Carnosic acid 0.29 : 1.29 : 0.1
B-carotene : Lutein : Carnosic acid 0.39 : 1.29 : 0.1
B-carotene : Lutein : Carnosic acid 0.32 : 3.42 : 0.1
B-carotene : Lutein : Carnosic acid 0.16 : 1.71 : 0.1
B-carotene : Lutein : Carnosic acid 0.29 : 1.29 : 0.1
B-carotene : Lutein : Carnosic acid 0.39 : 1.29 : 0.1

The various active components may be formulated for either system or topical use. In the case of systemic administration, the polyphenol(s) and carotenoid(s) may be incorporated into oral dosage forms such as tablets, caplets, capsules, syrups, elixirs, liquids etc.

In other preferred embodiments, the composition of the present invention may be administered topically, for example on the skin or mucous membranes (e.g. as creams, lotions, ointments etc.). Further details of suitable methods of incorporating the polyphenol and carotenoid-containing compositions of the present invention into the various different dosage forms may be obtained from any standard reference work known to the skilled artisan, including, for example, Remington's Pharmaceutical Sciences, Mack . Publishing Co, Easton, Pa, USA (1980).

In other preferred embodiments, the composition of the present invention is prepared as a food additive that is suitable for direct incorporation into a foodstuff or a beverage.

The carnosic acid used to prepare the compositions of the present invention may be obtained commercially from several different suppliers including Alexis Biochemicals, Lausen, Switzerland. The carotenoids were obtained from several different suppliers including LycoRed Ltd., Be'er Sheva, Israel.

Alternatively, some of the components of the composition, such as lycopene may be incorporated into said composition in the form of a lycopene-rich tomato extract. One such tomato extract is commercially available (e.g. in capsule form) from LycoRed Ltd., Beer Sheva, Israel under the trade name "Lyc-O-Mato®". Suitable processes for preparing this extract and similar extracts are described in US 5,837,311, the specification of which is incorporated herein by reference in its entirety. However, it is to be recognized that many other types of preparatory procedures may be used to obtain the carotenoid-containing composition from a variety of plant sources. In addition, the composition may also be prepared from one or more synthetic carotenoids.

The following examples are provided for illustrative purposes and in order to more particularly explain and describe the present invention. The present invention, however, is not limited to the particular embodiments disclosed in these examples.

### Example 1

### Inhibition of production of NO, TNF-alpha and PGE₂ in peritoneal macrophages by various combinations of carnosic acid, lutein, lycopene and beta-carotene

### Methods and materials:

*Macrophage isolation and cell culture -* Peritoneal macrophages were collected from the peritoneal cavity of 6-8 week old male ICR mice (Harlan, Israel) that had been given an intraperitoneal injection of 1.5 ml of thioglycollate broth (4%) 4 days before harvest. Peritoneal macrophages were washed three times with PBS and, if needed, a hypotonic lysis of erythrocytes was performed, yielding 90-95% purity. The macrophages were identified by FACS analysis using FITC-conjugated rat anti-mouse F4/80 (MCA497F) (Serotec, Oxford, England) by flow microfluorimetry on FACS (Becton Dickinson, Mountain View, CA). For each sample, 10,000, light scatter-gated viable cells were analyzed. Peritoneal macrophages and murine macrophage cell line RAW264.7 were cultured RPMI 1640 medium containing 10% FCS, 2 mM L-glutamine; 100 U/ml penicillin; 100 µg/ml streptomycin (Beit-Haemek, Israel) in 96-well plates (1 × 10⁶ cells/well) at 37°C in 5% CO₂ atmosphere. Cells were stimulated with LPS (0.1- 1 µg/ml) in the presence or absence of carnosic acid and/or one or more of the following carotenoids: carnosic acid, purified lycopene, lycopene-rich tomato extract (Lyc-O-Mato®; LycoRed Ltd., Be'er Sheva, Israel), lutein and beta-Carotene.

The carnosic acid and the various carotenoids were dissolved in DMSO (to a final concentration of 5mM). The mixture was vortexed and incubated in a water bath at 37°C (with shaking) for 10 min and then sonicated in a sonicator bath three times for 15 seconds each time. Using this stock solution the desired concentrations were prepared by the addition of appropriate volumes thereof to warm culture medium.

The concentration of lycopene in the solution was determined after extraction as follows: 0.5 ml isopropanol. + 1.5 hexane /dichloromethane (1:5 V/V) containing 0.025% BHT were added to 1 ml of lycopene solution freshly prepared at a concentration of 20uM in preheated medium,. The solution was vortexed and the phases were separated by centrifugation 3000 rpm for 10 min.

A spectrum analysis is conducted to measure the content of lycopene (absorption peak at 471 nm.)

Appropriate volumes of DMSO (0.1-0.2%) were added to the controls and the percent inhibition in each test tube was calculated in relation to its control.

*NO production assay -* NO levels in supernatants of cell cultures were determined by assaying nitrite levels using Griess reagent and sodium nitrite as a standard as described in Green, L. C., Wagner, D. A., Glogowski, J., Skipper, P. L., Wishnok, J. S., and Tannenbaum, S. R. (1982) Anal Biochem. 126: 131-138.

PGE₂ *measurement -* Supernatants of resting and stimulated cells were collected and immediately stored at -70°C. PGE₂ levels were determined by utilizing a dextran coated charcoal radioimmunoassay protocol as previously described (Dror N, Tveria L, Meniv I, Ben-Shmuel S, Filipovich T, Fleisher-Berkovich S., Regul Pept. 2008 150: 21-5).

Briefly, 100 µl sample or PGE₂ standard (Sigma Israel, Rehovot, Israel) were incubated in the presence of 500 µl anti-PGE₂ antiserum (Sigma Israel, Rehovot, Israel) for 30 min. [³H]PGE₂ (Amersham Biosciences, NJ, USA) was added next for 24 h at 4°C. 24 h later, 200 µl cold dextran coated charcoal suspension was added to each tube and incubated for 10 min on ice. The tubes were centrifuged at 3500 RPM for 15 min at 4°C. 500µl of supernatants containing [³H] PGE₂-anti-PGE₂ complexes were counted (Packard Spectrometry 1900CA) and the amount of PGE₂ was calculated.

*TNF-alpha production assay -* Concentrations of TNF-alpha were quantified using ELISA kits (Biolegend Inc., San Diego, CA).

*Statistical analysis -* Data are presented as the mean ± SEM. Statistical significance for comparisons between groups was determined using Student's paired two-tailed t test.

### RESULTS

### Fig. 1

### Dose dependent synergistic inhibition of NO production by combination of lycopene or Lycomato with Carnosic acid.

The results obtained using carnosic or individual carotenoids alone are as follows: Lycopene or Lycomato in the range of 1-5 µM caused low level inhibition of NO production. Carnosic acid (1 and 2 µM) caused 12% and 18% inhibition of NO production, respectively.

The addition of carnosic acid to lycopene or Lycomato caused a synergistic inhibition of NO production which was dose dependent.

Combination of Carnosic acid with Lycomato is more effective than with purified lycopene.

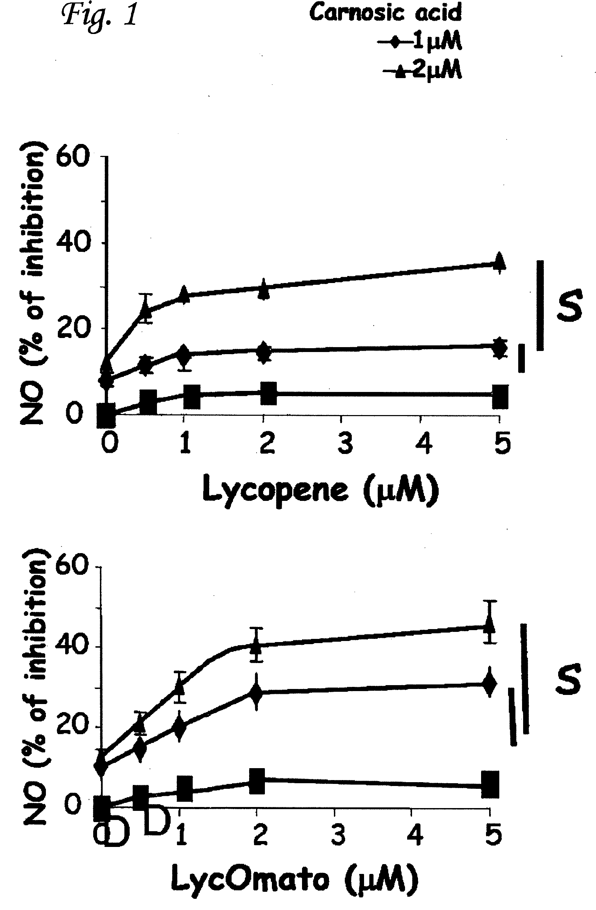

The results presented in Fig. 1 are the means±SEM of 10 independent experiments each in duplicates.

### Fig. 2a.

### Inhibition of NO production by combination of optimal low concentrations of two components: lycopene or Lycomato with carnosic acid, lutein and beta-carotene.

Combination of 1µM lycopene or Lycomato with 2 µM Carnosic acid caused significant synergistic inhibition of NO production, which was more effective in the presence of Lycomato compared with lycopene.

Combination of 1µM lycopene or Lycomato with 1 µM lutein or with 2 µM beta-carotene caused an additive or non significant synergistic inhibition of NO production, respectively.

Combination of lycopene or Lycomato with carnosic acid (i.e. a combination of a carotenoid with a polyphenol) is more effective than the combination of two cartenoids.

### Fig. 2b.

### Inhibition of NO production by combination of optimal low concentrations of lycopene or Lycomato with two other components.

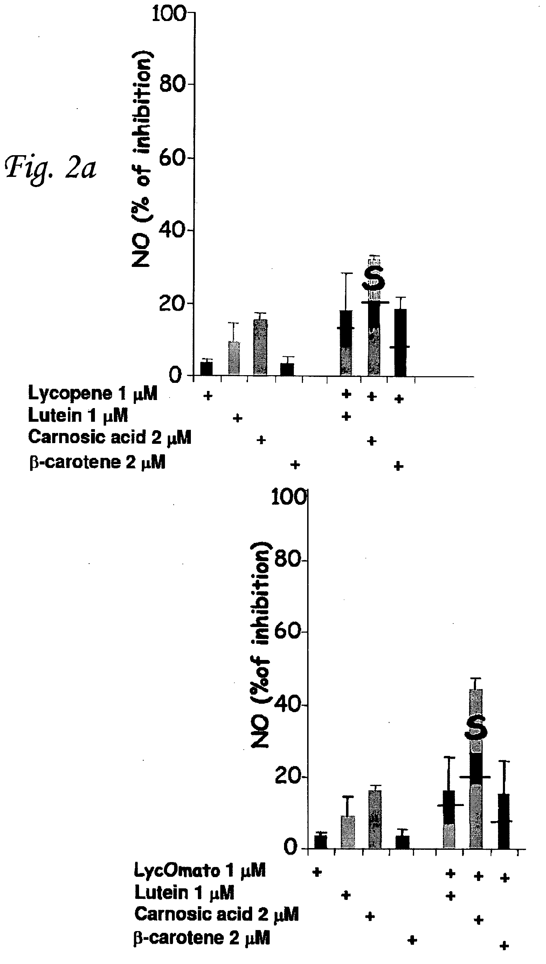

Combination of lycopene or Lycomato with carnosic acid and lutein or with carnosic acid and beta-Carotene (concentrations the same as used to generate the results shown in Fig. 2a) caused a significant and similar synergistic inhibition of NO production, while a combination excluding carnosic acid caused only an additive effect (marked with a dashed ellipse).

Combination of the four components (i.e. lycopene or Lycomato together with carnosic acid, lutein and beta-carotene did not improve upon the combination of the three components.

### Fig. 2C.

### Combination of carnosic acid and carotenoid excluding lycopene or Lycomato.

Combination of either lutein or beta-Carotene with carnosic acid caused a significant and similar synergistic inhibition of NO production (which is similar to the combination of lycopene and carnosic acid but lower than that seen with the combination of Lycomato and carnosic acid). Combination of lutein and beta-Carotene caused additive (or lower) effect.

These results further support that both cartenoid(s) and polyphenol(s) are required in order to obtain the optimal synergistic effect.

The results are the means±SEM of 3 independent experiments, each produced in duplicate.

### Fig. 3.

### Upper graph: Inhibition of TNF-alpha production by different combinations of optimal low concentrations of lycopene with carnosic acid, lutein and beta-carotene.

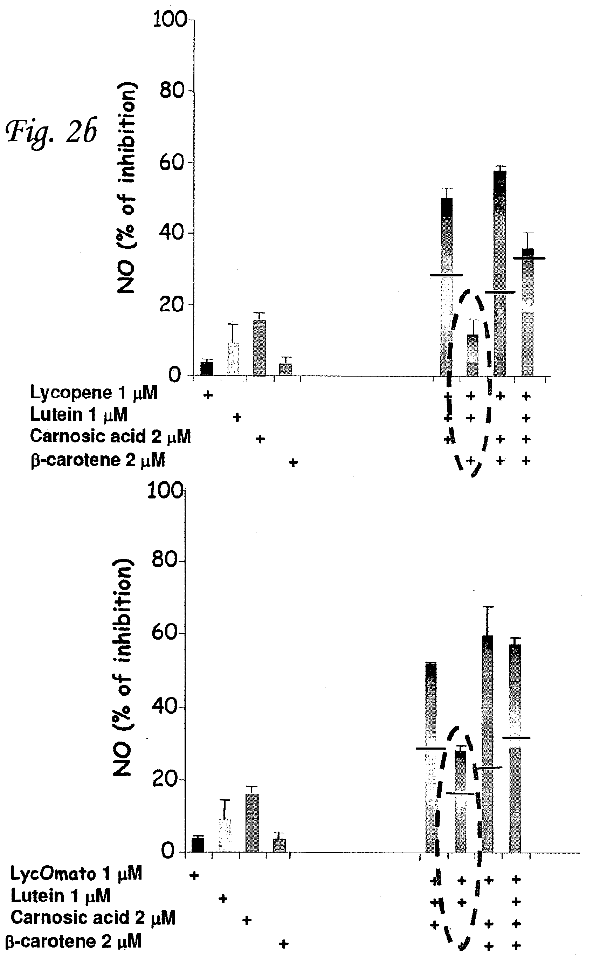

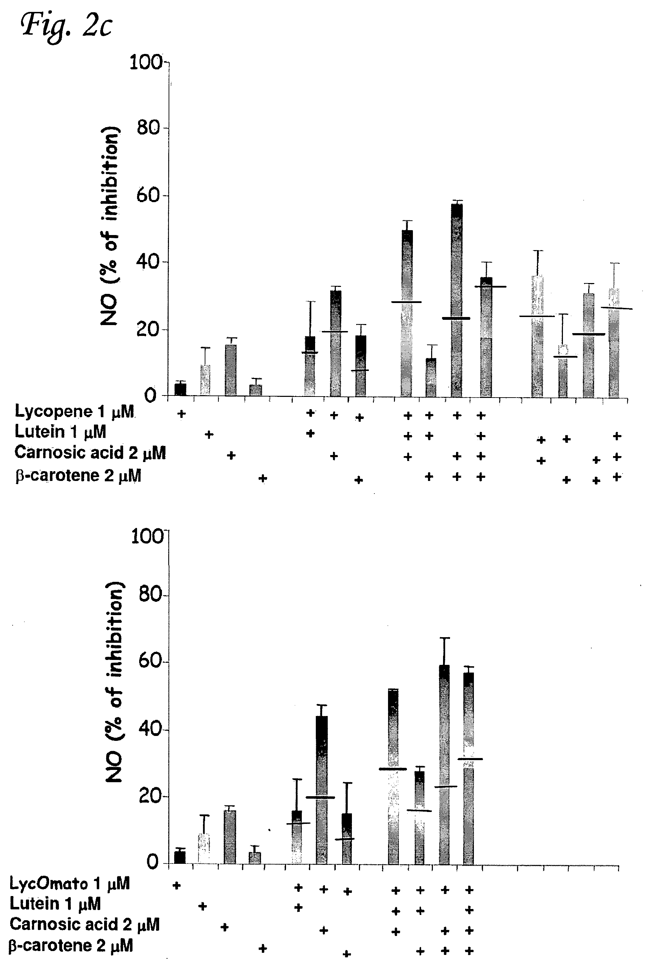

TNF-alpha production in the same set of experiments as in Figure 2 was less sensitive than NO production as none of these agents caused any detectable inhibition of TNF-alpha production when used alone (i.e. not in combination with other agents).

Combinations of lycopene with carnosic acid or with beta-carotene caused a low-level synergistic inhibition of TNF-alpha production: 10% and 8%, respectively.

Similar to the effect on NO production, combinations of lycopene with either carnosic acid and lutein or with carnosic acid and beta-carotene caused a significant and similar synergistic inhibition of TNF-alpha production, which was higher than the synergistic inhibition caused by combination excluding carnosic acid.

Combination of carnosic acid with all three carotenoids did not improve upon the synergistic result obtained with the aforementioned combination of carnosic acid with two carotenoids.

Lower graph: Inhibition of TNF-alpha production by different combinations of optimal low concentrations of Lycomato with carnosic acid, lutein and beta-carotene.

TNF-alpha production was inhibited (10%) in the presence of Lycomato (in contrast to the lack of detectable inhibition in the presence of lycopene). Combinations of Lycomato with each of the other carotenoids caused a synergistic inhibition that was higher in the presence of carnosic acid.

Similar to the effect on NO production, combinations of Lycomato with carnosic acid and lutein or with carnosic acid and beta-Carotene caused a significant and similar synergistic inhibition of TNF-alpha production, while a combination excluding carnosic acid caused a lesser synergistic effect.

As in the case of purified lycopene, a combination of carnosic acid with all three carotenoids did not improve upon the synergistic result obtained with the aforementioned combination of carnosic acid with two carotenoids.

The results are the means±SEM of 3 independent experiments, each performed in duplicate.

It is to be noted that combinations that included Lycomato were more effective in inhibiting TNF-alpha production than those that incorporated purified Lycopene.

### Fig. 4

### Inhibition of PGE₂ production by different combination of optimal low concentrations of Lycopene with Carnosic acid, Lutein and beta-carotene.

PGE₂ production in the same set of experiments as reported in Fig. 2 was more sensitive than NO production to carnosic acid or beta-Carotene when used alone (around 20% inhibition by each). Combinations of lycopene with lutein, carnosic acid or beta-Carotene caused a synergistic inhibition of PGE2 production.

A low level synergistic inhibition could be detected with a combination of Lycomato with lutein and carnosic acid only, while a combination with carnosic acid and beta-carotene caused only an additive effect. A combination with lutein and beta-carotene caused an additive inhibition of PGE2 production.

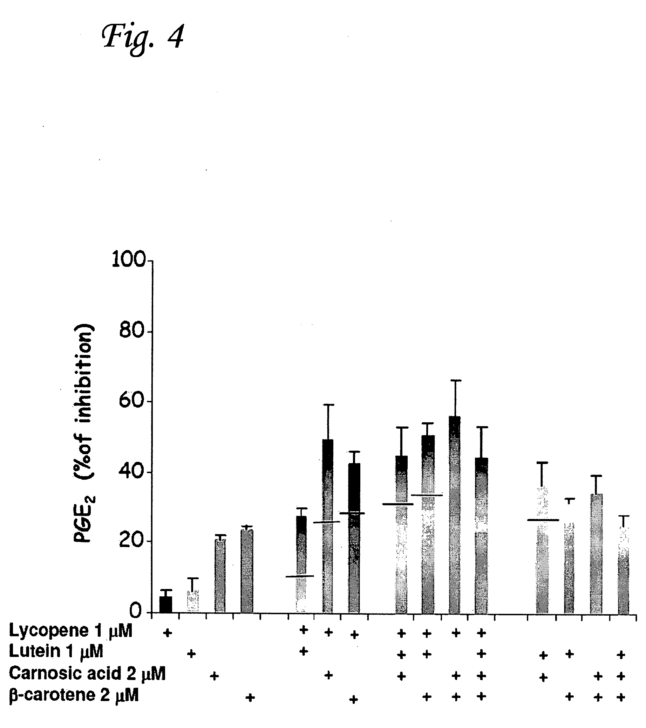

It will also be seen from Fig. 4 that, once more, a combination of four agents (carnosic acid plus three carotenoids) did not improve the combination of carnosic acid with two carotenoids.

### Fig. 5a

### Inhibition of PGE₂ production by different combinations of optimal low concentrations of Lycopene with carnosic acid, lutein and beta-carotene.

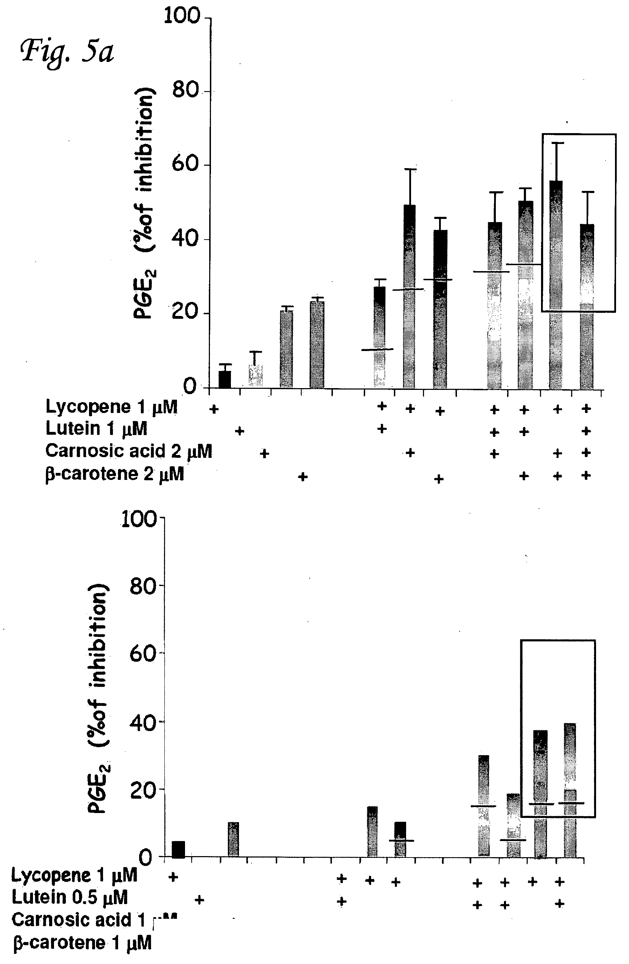

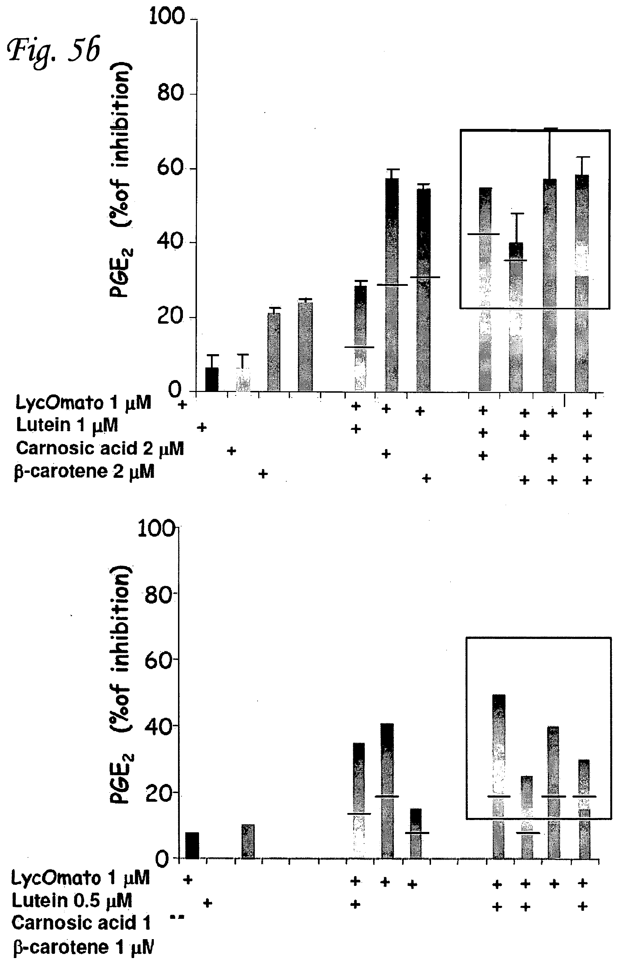

As already shown in Fig. 4, Fig. 5a upper panel shows that carnosic acid or beta-Carotene (each used separately) caused high-level inhibition of PGE2 production (around 20% inhibition by each). Combinations of lycopene with lutein, carnosic acid or beta-carotene caused a synergistic inhibition of PGE2 production.

A low-level synergistic inhibition could be detected in the case of a combination of lycopene with lutein and carnosic acid only, while a combination with carnosic acid and beta-Carotene caused only an additive effect. A combination containing lutein and beta-carotene caused additive inhibition of PGE₂ production. Consequently, lower concentrations were studied (as shown in Fig. 5a lower panel, discussed below).

A combination of all four active agents (i.e. carnosic acid plus three carotenoids) did not improve the combination of the carnosic acid with two carotenoids.

### Synergistic inhibition of PGE₂ production by different combination of optimal lower concentrations of carnosic acid, lutein and beta-carotene (Lower panel).

Neither lutein (0.5 µM) nor beta-Carotene (1 µM) alone affected PGE₂ production, while carnosic acid (1 µM) caused 10% inhibition. In these conditions combinations of lycopene 1mM with lower concentrations of either of two other components caused synergistic inhibition.

The combination of all four active agents did not improve the combination of the three.

### Fig. 5b

### inhibition of PGE₂ production by different combinations of optimal low concentrations of Lycomato with carnosic acid, lutein and beta-carotene.

The upper panel shows that the effect of Lycomato on inhibition of PGE2 production is similar to that of pure Lycopene and the similar combinations resulted with similar effect as shown for lycopene (Fig. 5a upper panel).

### Synergistic inhibition of PGE₂ production by different combination of optimal lower concentrations of carnosic acid, lutein and beta-carotene (Lower panel).

As in Fig 5a, neither lutein (0.5 µM) nor beta-Carotene (1 µM) alone affected PGE2 production, while carnosic acid (1 µM) caused 10% inhibition. The combination with beta-carotene was additive, while the combination with lutein or with carnosic acids were synergistic and much higher than that obtained with purified lycopene (Fig. 5a). The combination of Lycomato with carnosic acid and lutein or with carnosic acid and beta-Carotene caused a significant and similar synergistic inhibition of PGE2 production. A combination of lutein and beta-Carotene (excluding carnosic acid) caused a lower level of synergistic inhibition. At these concentrations, a combination of Lycomato with lutein and carnosic acid caused a synergistic effect which was much higher than that resulted from the use o.f combinations with purified lycopene.

A combination of all four active agents (i.e. carnosic acid plus three carotenoids) did not improve the combination of the carnosic acid with two carotenoids.

**Fig**. **6**

### Synergistic inhibition of NO production by combinations of lutein, beta-carotene and carnosic acid.

The upper two graphs (A and B) illustrate the synergistic interaction between the three components of the tested composition on NO production, wherein the final concentration of beta-carotene was 0.5 µM. Similarly, compositions containing a higher concentration of beta-carotene (1.0 µM; graphs C and D) also caused inhibition of NO production in a synergistic manner.

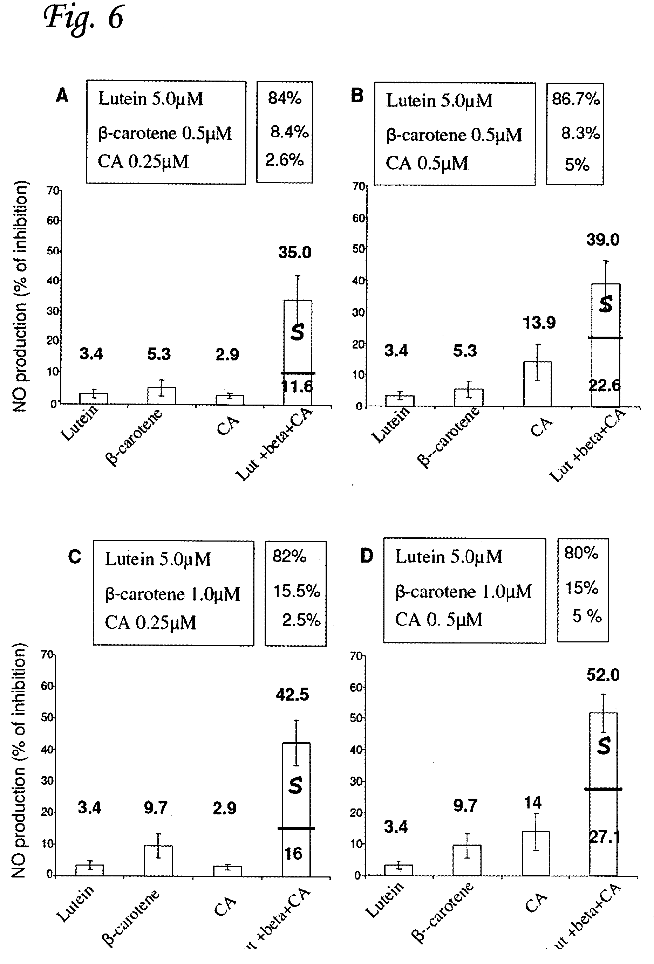

In each of the graphs presented in Fig. 6, the horizontal line in the bar corresponding to the three-component composition indicates the level of NO inhibition that would be expected if the effect of each of said components were additive. The greatly increased level of inhibition seen (the area of the bar above the horizontal line marked with an 'S') indicated that the three components of the composition acted synergistically.

### Fig. 7

### Synergistic inhibition of TNFα production by combinations of lutein, beta-carotene and carnosic acid.

The upper two graphs (A and B) illustrate the synergistic interaction between the three components of the tested composition on TNFα production, wherein the final concentration of beta-carotene was 0.5 µM. Similarly, compositions containing a higher concentration of beta-carotene (1.0 µM; graphs C and D) also caused inhibition of TNFα production in a synergistic manner.

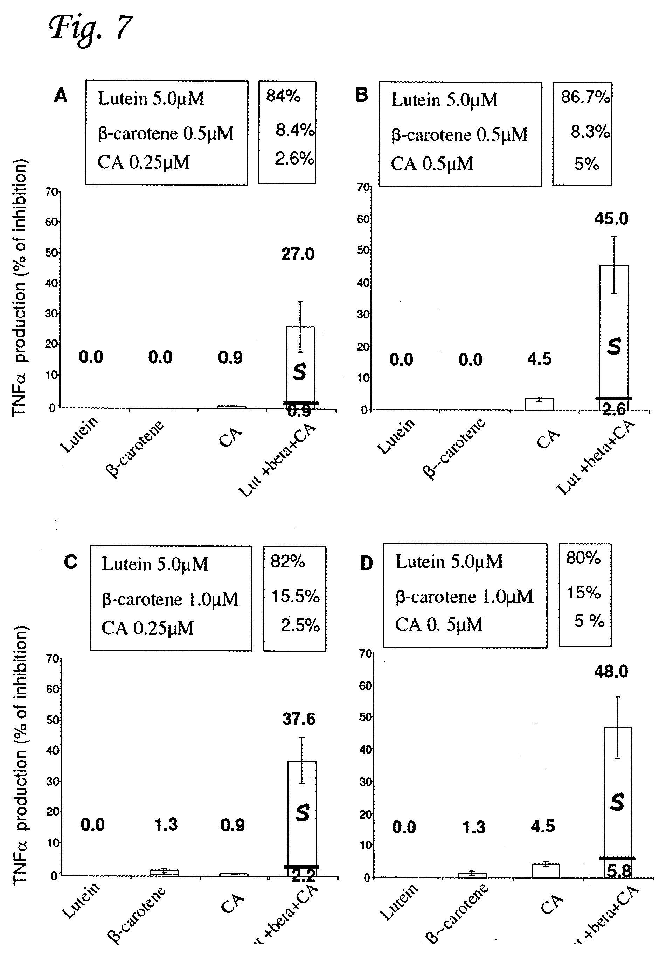

In each of the graphs presented in Fig. 7, the horizontal line in the bar corresponding to the three-component composition indicates the level of TNFα inhibition that would be expected if the effect of each of said components were additive. The greatly increased level of inhibition seen (the area of the bar above the horizontal line marked with an 'S') indicated that the three components of the composition acted synergistically.

### Example 2

### Inhibition of LPS-induced NO production in peritoneal macrophages by various combinations of lutein, lycopene and a polyphenol selected from the group consisting of carnosic acid, gallic acid, resveratrol and quercetin

### Methods and materials:

*Macrophage isolation and cell culture -* Peritoneal macrophages were collected and cultured as described in Example 1, hereinabove.

*Preparation of test agents -* Lycopene and Lutein were dissolved in DMSO (the volume of DMSO in the test solution did not exceed 0.04%). The mixture was vortexed and shaken at 37°C for 10 min and sonicated in a sonicator bath for 15 sec X 3 times. From this stock solution the desired concentrations were reached by addition of appropriate volumes to warm culture medium. The concentration in the solution was calculated to 1 ml of the highest final concentration 0.5 ml isopropanol+ 1..5 ml hexane / dichloromethane (1:5 V/V) containing 0.025% BHT. The solution was vortexed and the phases were separated by centrifugation at 3000 rpm for 10 min. A spectrum analysis was conducted to detect the level of nutrients. Carnosic acid, Resveratrol, Gallic acid or Quercetin were dissolved in ethanol (the volume of ethanol in the test solution did not exceed 0.0025%).

Appropriate volumes of DMSO and/or ethanol were added to the controls and the percent inhibition of each test tube was calculated in relation to its control tube.

*NO production assay -* NO levels in supernatants of cell cultures were determined by assaying nitrite levels using Griess reagent and sodium nitrite as described hereinabove in Example 1.

*Statistical analysis -* Data are presented as the mean ± SEM. Statistical significance for comparisons between groups was determined using Student's paired two-tailed t test.

### Results:

### Figure 8

### A. Synergistic inhibition of NO production by combinations of low concentrations of Lycopene, Lutein and each of the different polyphenols

Macrophages were incubated with 1µM Lycopene, 1µM Lutein and either 2 µM Carnosic acid, 2 µM Resveratrol, 2 µM Gallic acid or 2 µM Quercetin and their combinations for 1 h before addition of LPS for 16h at 37°C. NO production was measured and the % of inhibition was calculated. In each experiment the effect of three different concentrations of LPS is analyzed, as the sensitivity of the cells may change in different experiments.

Combinations of 1µM Lycopene, 1µM Lutein and 2 µM of either Carnosic acid Resveratrol, Gallic acid or Quercetin, caused a significant synergistic inhibition of NO production (indicated by the letter "S" in the graph, wherein the horizontal line crossing each of the bars representing the synergistic combinations indicates the results to be expected if the interaction were additive and not synergistic) There were no significant differences between each of these various combinations. As shown in the Figure, the effect of each of the carotenoid or polyphenol tested at the given concentration was very low. As shown by the horizontal line in each graph, the synergistic effect was around three fold higher than that of the additive effect.

### B. Synergistic inhibition of NO production by combination of low concentrations of Lyc-O-Mato, Lutein and each of the different polyphenols.

Macrophages were treated as in A, but the experiments were conducted using Lyc-O-Mato instead of Lycopene.

Although Lyc-O-Mato by itself caused a similar inhibition of NO production as that caused by Lycopene, combinations with Lyc-O-Mato were more effective and resulted in higher synergism of about four fold compared with the additive effect.

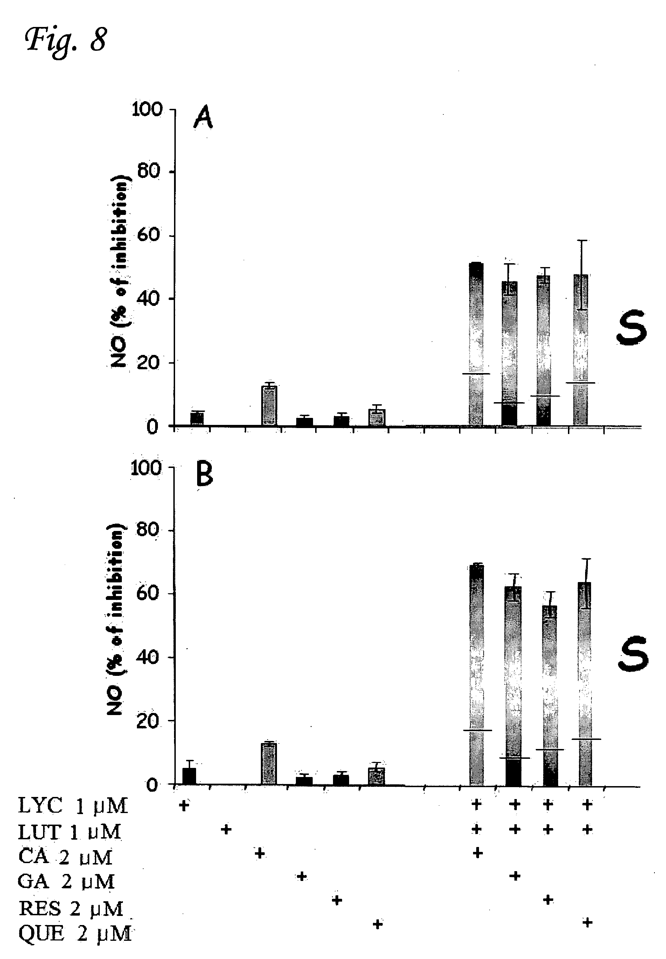

The results shown in Fig. 8 are shown as the means±SEM of three different experiments each done in triplicate.

### Example 3

### Inhibition of LPS induced superoxide production in macrophages by various combinations of lycopene or Lyc-O-Mato, lutein, beta-carotene and carnosic acid

### Methods and materials:

*Macrophage isolation:* Peritoneal macrophages were isolated and treated as described hereinabove in Example 1.

*Superoxide production:* The production of superoxide anion (O₂⁻) by macrophages was measured as the superoxide dismutase-inhibitable reduction of ferricytochrome c by the microtiter plate technique, as known in the prior art. An aliquot of radiolabelled macrophages (5 × 10⁵ cells/well) used for the adherence assay was taken and suspended in 100 µl incubation medium containing ferricytochrome c (150 mM). Stimulation was induced with PMA (50 ng/ml). The reduction of ferricytochrome c was followed by a change of absorbance at 550 nm at 2 min intervals for 30 min on a Thermomax Microplate Reader (Molecular Devices, Melno Park, Calif., USA). The maximal rates of superoxide generation were determined and expressed as nanomoles O₂⁻/10^{6.} cells/ 10 min using the extinction coefficient E₅₅₀ = 21 m*M*⁻¹ cm⁻¹.

### Results:

### Fig. 9

### Upper graph (A): Inhibition of superoxide production by different combinations of optimal low concentrations of p urified lycopene with carnosic acid, lutein or beta-carotene.

Superoxide production was inhibited in the presence of 2µM beta-carotene (10%).

Combinations of lycopene with carnosic acid or with beta-carotene caused a low-level inhibition of superoxide production that was not significantly different from the effect of beta-carotene.

Similar to the effect on NO production (see Example 1, hereinabove), combinations of lycopene with either carnosic acid and lutein, or with carnosic acid and beta-carotene caused a significant and similar synergistic inhibition of superoxide production, which was higher than the synergistic inhibition caused by a combination excluding carnosic acid.

Combination of carnosic acid with all three carotenoids did not improve upon the synergistic result obtained with the aforementioned combination of carnosic acid with two carotenoids.

### Lower graph (B): Inhibition of superoxide production by different combinations of optimal low concentrations of Lyc-O-Mato with carnosic acid, lutein or beta-carotene.

Superoxide production was inhibited (7%) in the presence of Lyc-O-Mato (in contrast to the lack of detectable inhibition in the presence of lycopene). Combinations of Lyc-O-Mato with each of the other carotenoids caused a caused a low-level inhibition of superoxide production that was not significantly different from the effect of beta-carotene or Lyc-O-Mato.

Similar to the effect on NO production (see Example 1, hereinabove), combinations of Lyc-O-Mato with carnosic acid and lutein or with carnosic acid and beta-Carotene caused a significant and similar synergistic inhibition, of superoxide production, while a combination excluding carnosic acid caused a lesser synergistic effect.

As in the case of purified lycopene, a combination of carnosic acid with all three carotenoids did not improve upon the synergistic result obtained with the aforementioned combination of carnosic acid with two carotenoids.

The results are the means±SEM of 3 independent experiments, each performed in duplicate.

### Example 4

### Inhibition of LPS induced p65-NFkB phosphorylation on Serine 536 in cell nuclear lysates and of iNOS and COX2 up-regulation by various combinations of lycopene or Lyc-O-Mato with lutein and carnosic acid

### Introduction

Expression of inflammatory cytokines as well enzyme protein expression can be regulated by the activation of the transcription factor nuclear factor-kappa B (NFκB), which is critically involved in several aspects of the pathogenesis chronic inflammatory diseases. NFκB is activated as a consequence of phosphorylation, ubiquitination, and subsequent proteolytic degradation'of the IκB protein through activation of IκB kinase (IKK). The liberated NFκB translocates into nuclei and binds to motifs in the promoters of pro-inflammatory genes such as inducible nitric oxide synthase (iNOS) and of cyclooxygenase 2 (COX2) TNF-α, and IL-1β, leading to the induction of their mRNA expression. Most of the anti-inflammatory drugs have been shown to suppress the expression of these genes by inhibiting the NFκB activation pathway. Thus, an NFκB inhibitor may be useful as a potential therapeutic drug in clinical applications for regulating the inflammation associated human diseases.

p65 NFκB RelA can be phosphorylated by PKA on Ser-276 or by a redox-sensitive mechanism on Ser-536. It has been shown that reactive oxygen species (ROS) plays an important role in NF-κB activation and inflammatory gene expression.

The aim of this study was to investigate whether low concentrations of the combinations of Lycopene/Lyc-O-Mato + Lutein + carnosic acid can cause a synergistic inhibition of NFκB activation.

NFκB activation was analyzed by its two phosphorylated forms: PKA dependent Ser-276 and redox-sensitive Ser-536.

### Methods

*Macrophage isolation:* Peritoneal macrophages were isolated and treated as described hereinabove in Example 1.
For detection of NF-kB activation the cell were treated with LPS for 10 min.

*Preparation of nuclear protein extract -* 2 × 10⁶ cells were suspended in 600 µl of ice-cold NP-40 lysis buffer (0.1% NP-40, 10 mM Tris-HCl, pH 7.4, 10 mM NaCl, 3 mM MgCl2, 1 mM EDTA, 10 µg/ml leupeptin, 10 µg/ml aprotonin, and 1 mM PMSF). The cells were vortexed for 15 s, kept on ice for 5 min, and centrifuged at 300 g for 10 min at 4°C. The resulting pellets (the nuclei containing fractions) were then immediately solubilized in electrophoresis sample buffer Nuclear integrity was verified directly by light microscopy, which also revealed that intact cells were rarely observed in nuclei-containing fraction (<2%).

*Total Cell lysates:* were prepared using 1% Triton X-100, 50 mM HEPES (pH 7.5), 150 mM NaCl, 1 mM EDTA, 1 mM EGTA, 10 % glycerol, 25 mM NaF, 10 µM ZnCl2, 1mM PMSF, and 100 µM leupeptin.

*Immunoblot analysis:* lysate proteins (35-50 µg) were separated by electrophoresis on 7.5% polyacrylamide SDS gels. The resolved proteins were electrophoretically transferred to nitrocellulose, which was stained with Ponsue red to detect protein banding, and then blocked in 5% milk in TBS (10 mM Tris,135 mM NaCl, pH 7.4). Immunoblot determination was done as described before (17) using primary antibodies p-P65, COX-2 and iNOS (Cell Signaling Technology, Beverly, MA) for overnight incubation at 4°C and second antibody, peroxidase conjugated goat antirabbit or antimouse (Amersham Biosciences, Buckinghamshire, United Kingdom) for 1 hour at room temperature and developed using the enhanced chemiluminescence (ECL) detection system (Amersham Biosciences).

For immunoblot detection of P-65 the nuclei fractions of 2 × 10⁶ cells were immediately solubilized in electrophoresis sample buffer and processed for separation on 8% SDS polyacrylamide gel electrophoresis (SDS-PAGE).

### Results

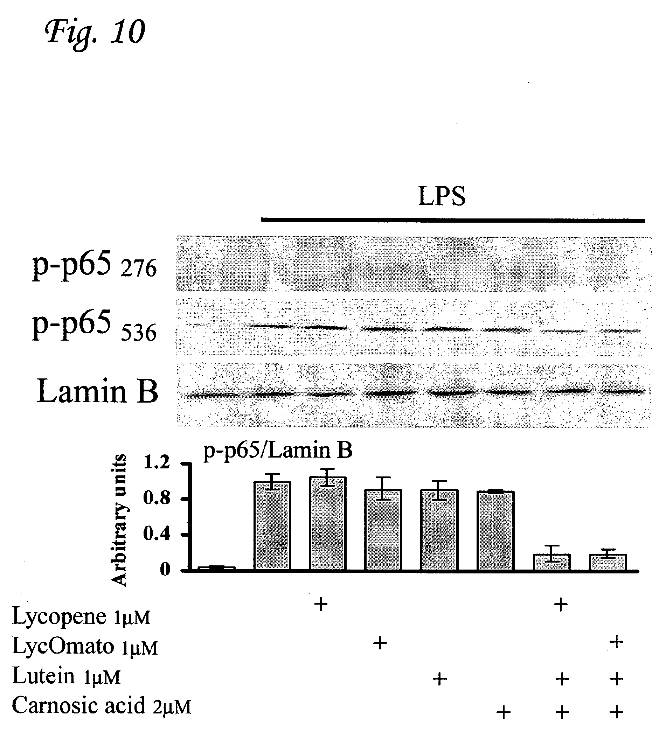

As shown in Fig. 10, in the representative immunoblot analysis, addition of combination of 1 µM lycopene or 1 µM Lyc-O-Mato with 1 µM lutein and 2 µM carnosic acid to peritoneal macrophages for 1 h before addition of LPS for 10 min caused a significant synergistic inhibition (of about 80%) of p65-NFκB phosphorylation on Serine 536 in cell nuclear lysates, while each nutrient alone had no effect at all. The intensity of each phospho p-65-NFκB band was divided by the intensity of each lamin band after quantitation by densitometry, and expressed as arbitrary units. Shown are the means±SEM of three independent experiments.

As shown in the immunoblot, there was no phosphorylation of p65 NFκB on Serine 276.

These results demonstrate that the carotenoid-polyphenol composition tested causes significant synergistic inhibition of NFκB activation mediated by phophorylation Ser-536 that is mediated by a redox-sensitive mechanism.

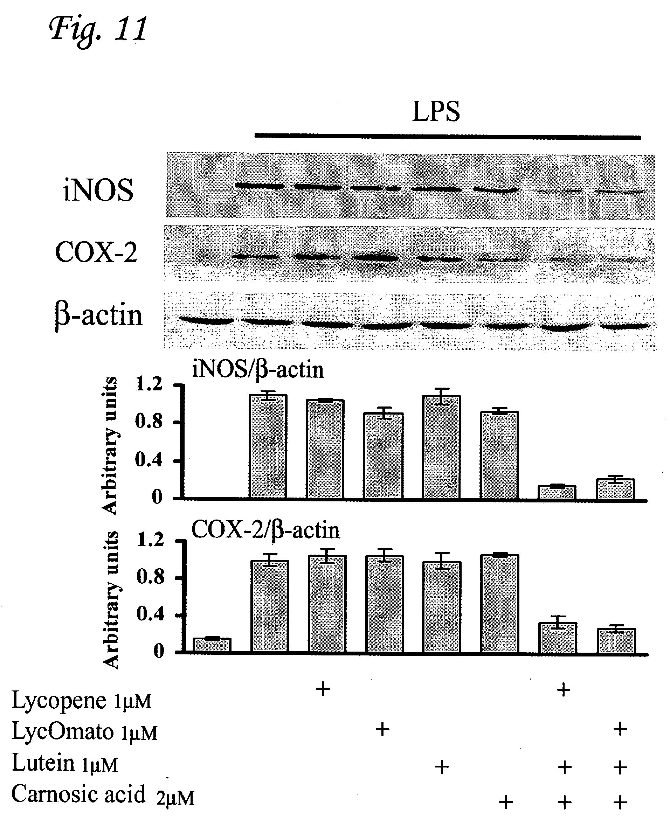

Under the same conditions, addition of the nutrient combinations (in concentrations detailed above) 1 h prior to addition of LPS for 24 h, caused a significant inhibition of iNOS and of COX2 protein expression in total cell lysates (Fig. 11). Each nutrient alone did not cause inhibition of the induction of either iNOS or COX2.

The intensity of each protein band (iNOS or COX2) was divided by the intensity of each β-actin band after quantitation by densitometry, and expressed as arbitrary units. Shown are the means±SEM of three independent experiments.

These results demonstrate a synergistic inhibition of the induction of both inflammatory enzymes at levels of around 80% and around 60% for iNOS and COX-2, respectively, and provide both support for the inhibition of NO production and PGE₂ release reported hereinabove, as well as a molecular explanation therefor.

While specific embodiments of the invention have been described for the purpose of illustration, it will be understood that the invention may be carried out in practice by skilled persons with many modifications, variations and adaptations, without departing from its spirit or exceeding the scope of the claims.

The following paragraphs describe various embodiments of the invention but are not to be taken as claims.
1. A therapeutic composition comprising one or more polyphenols and two or more carotenoids selected from the group consisting of lutein, lycopene and beta-carotene.
2. The therapeutic composition according to paragraph 1, wherein the one or more polyphenols are selected from the group consisting of carnosic acid, quercetin, resveratrol, gallic acid, chicoric acid, gingerol and curcumin.
3. The therapeutic composition according to paragraph 2, wherein the polyphenol is carnosic acid.
4. The therapeutic composition according to paragraph 2, wherein the polyphenol is quercetin.
5. The therapeutic composition according to paragraph 2, wherein the polyphenol is resveratrol.
6. The therapeutic composition according to paragraph 2, wherein the polyphenol is gallic acid.
7. The therapeutic composition according to paragraph 2 consisting essentially of lycopene, lutein and carnosic acid.
8. The therapeutic composition according to paragraph 2, consisting essentially of lutein, beta-carotene and carnosic acid.
9. The therapeutic composition according to paragraph 2, consisting essentially of lycopene, beta-carotene and carnosic acid.
10. The therapeutic composition according to any one of paragraphs 7- 9, wherein said composition further comprises phytoene .
11. The therapeutic composition according to any one of paragraphs 7- 9, wherein said composition further comprises phytofluene.
12. The therapeutic composition according to any one of paragraphs 7- 9, wherein said composition further comprises phytoene and phytofluene.
13. A method for inhibiting or reducing the production of superoxide ions, NO, TNF-alpha and/or PGE₂ in a mammalian subject comprising administering to said subject a therapeutic composition according to any one of paragraphs 1 to 12.
14. A method of treatment of pathological conditions in which, superoxide ions, NO, TNF-alpha and/or PGE₂ acts as a modulator or mediator of said condition in a mammalian subject in need of such treatment, wherein said method comprises administering to said subject a therapeutic composition according to any one of paragraphs 1 to 12.
15. The method of treatment according to paragraph 14, wherein the condition to be treated is an inflammatory condition.
16. The method of treatment according to paragraph 14, wherein the condition to be treated is selected from the group consisting of rheumatoid arthritis, adult respiratory distress syndrome (ARDS), asthma, rhinitis, idiopathic pulmonary fibrosis, peritonitis, cardiovascular inflammation, myocardial ischemia, reperfusion injury, atherosclerosis, sepsis, trauma, diabetes type II, retinopathy, psoriasis, gastrointestinal inflammation, cirrhosis, peritonitis and inflammatory bowel disease, and
17. The method according to any one of paragraphs 13 to 16 wherein the mammalian subject is a human subject.
18. The method according to any one of paragraphs 13 to 16, wherein the therapeutic composition is administered in a pharmaceutical dosage form.
19. The method according to any one of paragraphs 13 to 16, wherein the therapeutic composition is incorporated into a foodstuff or beverage.
20. Use of a combination of one or more polyphenols and two or more carotenoids selected from the group consisting of lutein, lycopene and beta-carotene in the manufacture of a medicament for the treatment of conditions responsive to inhibition of the production of superoxide ions, NO, TNF-alpha and/or PGE₂.
21. The use according to paragraph 20, wherein the one or more polyphenols are selected from the group consisting of carnosic acid, quercetin, resveratrol, gallic acid, chicoric acid, gingerol and curcumin.
22. The use according to paragraph 20, wherein the polyphenol is carnosic acid.
23. The use according to paragraph 20, wherein the polyphenol is quercetin.
24. The use according to paragraph 20, wherein the polyphenol is resveratrol.
25. The use according to paragraph 20, wherein the polyphenol is gallic acid.
26. The use according to paragraph 20, wherein the condition is an inflammatory condition.
27. The use according to paragraph 20, wherein the condition to be treated is selected from the group consisting of rheumatoid arthritis, adult respiratory distress syndrome (ARDS), asthnla, rhinitis, idiopathic pulmonary fibrosis, peritonitis, cardiovascular inflammation, myocardial ischemia, reperfusion injury, atherosclerosis, sepsis, trauma, diabetes type II, retinopathy, psoriasis, gastrointestinal inflammation, cirrhosis, peritonitis and inflammatory bowel disease, and neurodegenerative diseases, including Alzheimer's disease.
28. The use according to paragraph 20, wherein carnosic acid is used in combination with lycopene and lutein.
29. The use according to paragraph 20, wherein carnosic acid is used in combination with lycopene and beta-carotene.
30. The use according to paragraph 20, wherein carnosic acid is used in combination with lutein and beta-carotene.

## Claims

1. A therapeutic composition comprising one or more polyphenols and two or more carotenoids selected from the group consisting of lutein, lycopene and beta-carotene, wherein the polyphenols are selected from the group consisting of resveratrol, curcumin, quercetin, gallic acid, gingerol and chicoric acid.

2. The therapeutic composition according to claim 1, wherein the polyphenol is resveratrol.

3. The therapeutic composition according to claim 1, wherein the polyphenol is curcumin.

4. The therapeutic composition according to claim 1, wherein the polyphenol is quercetin.

5. The therapeutic composition according to claim 1, wherein the polyphenol is gallic acid.

6. The therapeutic composition according to claim 1, wherein the polyphenol is gingerol.

7. The therapeutic composition according to claim 1, wherein the polyphenol is chicoric acid.

8. The therapeutic composition according to any preceding claim, wherein the therapeutic composition consists essentially of lycopene, lutein and one of the polyphenols.

9. The therapeutic composition according to any preceding claim, consisting essentially of lutein, beta-carotene and one of the polyphenols.

10. The therapeutic composition according to any preceding claim, consisting essentially of lycopene, beta-carotene and one of the polyphenols.

11. The therapeutic composition according to any preceding claim, wherein said composition further comprises phytoene, phytofluene, or phytoene and phytofluene.

12. The therapeutic composition according to any preceding claim, for use in treating pathological conditions in which, superoxide ions, NO, TNF-alpha and/or PGE₂ acts as a modulator or mediator of said condition by inhibiting or reducing the production of superoxide ions, NO, TNF-alpha and/or PGE₂ in a mammalian subject, optionally wherein the mammalian subject is a human subject.

13. The therapeutic composition for use according to claim 12, wherein the condition to be treated is an inflammatory condition, optionally wherein the condition to be treated is selected from the group consisting of rheumatoid arthritis, adult respiratory distress syndrome (ARDS), asthma, rhinitis, idiopathic pulmonary fibrosis, peritonitis, cardiovascular inflammation, myocardial ischemia, reperfusion injury, atherosclerosis, sepsis, trauma, diabetes type II, retinopathy, psoriasis, gastrointestinal inflammation, cirrhosis, peritonitis and inflammatory bowel disease, and neurodegenerative diseases, including Alzheimer's disease.

14. The therapeutic composition for use according to claim 12 or 13, wherein the therapeutic composition is administered in a pharmaceutical dosage form.

15. The therapeutic composition for use according to any one of claims 12 to 14, wherein the therapeutic composition is incorporated into a foodstuff or beverage.
